(19) Europäisches Patentamt / European Patent Office / Office européen des brevets

(11) **EP 4 248 846 B1**

(12) **EUROPEAN PATENT SPECIFICATION**

(45) Date of publication and mention of the grant of the patent:
**07.08.2024 Bulletin 2024/32**

(21) Application number: **23164041.8**

(22) Date of filing: **24.03.2023**

(51) International Patent Classification (IPC):
**A61B 5/00** (2006.01)    **A61B 5/055** (2006.01)
**A61B 5/145** (2006.01)

(52) Cooperative Patent Classification (CPC):
**A61B 5/0075; A61B 5/055; A61B 5/14546; A61B 5/7264**

(54) **METHOD AND APPARATUS FOR NON-INVASIVE QUANTIFICATION OF METABOLITES IN THE BODY BASED ON ARTIFICIAL NEURAL NETWORKS**

VERFAHREN UND VORRICHTUNG ZUR NICHT-INVASIVEN QUANTIFIZIERUNG VON STOFFWECHSELPRODUKTEN IM KÖRPER AUF DER GRUNDLAGE VON KÜNSTLICHEN NEURONALEN NETZEN

MÉTHODE ET APPAREIL DE QUANTIFICATION NON INVASIVE DES MÉTABOLITES DANS L'ORGANISME BASÉS SUR DES RÉSEAUX NEURONAUX ARTIFICIELS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **25.03.2022 KR 20220037208**
**09.05.2022 KR 20220056351**

(43) Date of publication of application:
**27.09.2023 Bulletin 2023/39**

(73) Proprietor: **Metlit, Inc.**
**Seoul 03170 (KR)**

(72) Inventor: **LEE, Hyeong Hun**
**10583 Goyang-si, Gyeonggi-do (KR)**

(74) Representative: **BCKIP Part mbB**
**Siegfriedstraße 8**
**80803 München (DE)**

(56) References cited:
- **HYEONGHUN LEE AND HYEONJIN KI: "Bayesian deep learning-based 1H-MRS of the brain: Metabolite quantification with uncertainty estimation using Monte Carlo dropout", PROCEEDINGS OF THE 2021 ISMRM & SMRT ANNUAL MEETING & EXHIBITION, 15-20 MAY 2021, ISMRM, 2030 ADDISON STREET, 7TH FLOOR, BERKELEY, CA 94704 USA, no. 2014, 30 April 2021 (2021-04-30), XP040724033**
- **LEE HYEONG HUN ET AL: "Deep learning-based target metabolite isolation and big data-driven measurement uncertainty estimation in proton magnetic resonance spectroscopy of the brain", MAGNETIC RESONANCE IN MEDICINE, vol. 84, no. 4, 5 March 2020 (2020-03-05), US, pages 1689 - 1706, XP055860640, ISSN: 0740-3194, Retrieved from the Internet <URL:https://onlinelibrary.wiley.com/doi/full-xml/10.1002/mrm.28234> DOI: 10.1002/mrm.28234**

**Description**

BACKGROUND

**1. Field of the Invention**

The present disclosure relates to a computer-implemented method and apparatus, as well as a non-transitory computer-readable storage medium, for non-invasively quantifying metabolites in the body using an artificial neural network technology.

**2. Discussion of Related Art**

In general, magnetic resonance spectroscopy (MRS) is used for non-invasive quantification of metabolites in the body. MRS data is a resonance signal of a one-dimensional data type that may be acquired from a magnetic resonance imaging (MRI) system for the whole body. In the MRS data, resonance signals of various metabolites including water may appear by overlapping at positions in a specific anatomical region.

However, the quality of the resonance signal significantly degrades compared to magnetic resonance image data due to the patient's motion, hardware performance, and the characteristics of scan parameters. Since the metabolites in the body contain various types of pathophysiological information of patients and have the potential to be used for medical diagnosis, it is very important to separate and quantify the resonance signals of metabolites in MRS data without information distortion.

A paper by HYEONGHUN LEE AND HYEONJIN KI: "Bayesian deep learning-based 1H-MRS of the brain: Metabolite quantification with uncertainty estimation using Monte Carlo dropout", PROCEEDINGS OF THE 2021 ISMRM & SMRT ANNUAL MEETING & EXHIBITION, 15-20 MAY 2021, ISMRM, 2030 ADDISON STREET, 7TH FLOOR, BERKELEY, CA 94704 USA, no. 2014, 30 April 2021 (2021-04-30), XP040724033 focuses on the training of the BCNN (Bayesian convolutional neural network) and the estimation of metabolite content and corresponding uncertainty. The metabolite content is estimated by performing multiple regression using the metabolite basis set and a predictive mean spectrum, which is the mean spectrum over the BCNN-predicted metabolite-only spectra. The uncertainty in metabolite content is estimated by performing multiple regression using a 2SD (two-standard deviation) spectrum. The 2SD spectrum is obtained from the total uncertainty spectrum, which is calculated as the sum of an aleatoric uncertainty and an epistemic uncertainty. A metabolite basis set is used in absolute mode when estimating uncertainty in accordance with the 2SD spectrum.

SUMMARY OF THE INVENTION

The invention is set out in the appended set of claims.

Summary of the disclosure

The object of the present invention is to provide a computer-implemented method and apparatus, as well as a non-transitory computer readable storage medium for non-invasive quantification of metabolites in the body capable of improving the accuracy of metabolites quantification and the uncertainty information of metabolites quantification compared to the conventional method by constructing a Bayesian convolutional neural network (BCNN)-based variational inference network optimized for a magnetic resonance spectroscopy (MRS) data format and developing a pre-processing algorithm and post-processing algorithm of the network.

The technical problems/objects to be solved by the present disclosure are not limited to the above-mentioned technical problems. That is, other technical problems/objects that are not mentioned may be obviously understood by those skilled in the art to which the present disclosure pertains from the following detailed description. The above object is solved by the attached independent claims and further embodiments and improvements of the invention are listed in the attached dependent claims. Hereinafter, up to the "brief description of the drawings", expressions like "... aspect according to the invention", "according to the invention", or "the present invention", relate to technical teaching of the broadest embodiment as claimed with the independent claims. Expressions like "implementation", "design", "optionally", "preferably", "scenario", "aspect" or similar relate to further embodiments as claimed, and expressions like "example", "...aspect according to an example", "the disclosure describes", or "the disclosure" describe technical teaching which relates to the understanding of the invention or its embodiments, which, however, is not claimed as such.

The invention provides a computer-implemented method for non-invasive quantification of metabolites in a body including predicting aleotoric uncertainty information and a sample spectrum from a pre-processed input spectrum using an artificial neural network, wherein the sample spectrum has a form in which signal quality is improved compared to the pre-processed input spectrum, generating a predictive mean spectrum by calculating an average of the sample spectrum, calculating an epistemic uncertainty spectrum of the sample spectrum based on a variance of the sample spectrum, calculating an aleatoric uncertainty spectrum of the sample spectrum based on an average of the aleatoric uncertainty information, and calculating a two-standard deviation spectrum (2SD spectrum) of the sample spectrum based on the epistemic uncertainty spectrum and the aleatoric uncertainty spectrum.

The artificial neural network may include a Bayesian convolutional neural network (BCNN).

When compared to the pre-processed input spectrum, in the sample spectrum, signal-to-noise ratio (SNR), linewidth, and phase/frequency may be adjusted.

The predicting may include predicting T sample spectra and T pieces of aleatoric uncertainty information per the pre-processed input spectrum through T-time Monte Carlo dropout sampling (MCDO sampling), the sample spectrum may correspond to a metabolite-only output spectrum, and the aleatoric uncertainty information may correspond to a noise variance spectrum.

The predictive mean spectrum may be generated based on an average value of the sample spectra for each data point.

The calculating of the 2SD spectrum may include calculating a total uncertainty spectrum by summing the epistemic uncertainty spectrum and the aleatoric uncertainty spectrum, calculating a standard deviation spectrum (SD-spectrum) from a 1/2 power of the total uncertainty spectrum, and calculating the 2SD spectrum by multiplying the SD spectrum by 2.

According to the invention, the method according to the invention further includes performing baseline correction on the predictive mean spectrum and the 2SD spectrum, calculating a metabolite content value by performing linear regression on the corrected predictive mean spectrum, calculating a metabolite standard deviation value by performing linear regression on the corrected 2SD spectrum, and normalizing the metabolite content value and the metabolite standard deviation value.

The method may further include converting the normalized metabolite content value and the normalized metabolite standard deviation value into a vector form and transmitting the converted vector to an output unit.

According to the invention, the normalizing is based on (1) a relative content of the metabolite to water or (2) a relative content of the metabolite to a reference metabolite.

According to the invention, the relative content of the metabolite to the water is based on Equation 1.

[Equation 1]
$$Conc_{meta} = 55 * \left(\frac{S_{meta}}{S_{water}}\right) * CorrTiss * \frac{Nspins_{water}}{Nspins_{meta}},$$

where $Conc_{meta}$ denotes a content of a target metabolite (mmol/L), the $S_{meta}$ denotes a signal area of the target metabolite, the $S_{water}$ denotes a signal area of the water, the CorrTiss denotes a ratio of water in a biological tissue from which a MRS signal is obtained, the $Nspins_{water}$ denotes the number of spins of water participating in generating a resonance signal, and the $Nspins_{meta}$ denotes the number of spins of the target metabolite participating in generating the resonance signal.

The method may further include forming a reconstructed signal by calculating a difference between the normalized metabolite content value and the predictive mean spectrum, forming a reconstructed signal by calculating a difference between the normalized metabolite standard deviation value and the 2SD spectrum, and checking accuracy of the normalization based on a result of comparing the reconstructed signals.

The method may further include transmitting the predictive mean spectrum and the 2SD spectrum to an output unit.

According to the invention, there is also provided an apparatus for non-invasive quantification of metabolites in a body, which includes: one or more processors; and one or more memories configured to store instructions that, when executed by the one or more processors, cause the one or more processors to perform a calculation, in which the calculation performed by the one or more processors may include a calculation of pre-processing encrypted MRS data to provide an input spectrum; a calculation of predicting aleatoric uncertainty information and a sample spectrum from the pre-processed input spectrum using an artificial neural network, a calculation of calculating a predictive mean spectrum of the predicted sample spectrum and a 2SD spectrum of the predicted sample spectrum based on the predicted sample spectrum and the predicted aleatoric uncertainty information, a calculation of calculating a metabolite content value by performing linear regression on the predictive mean spectrum, a calculation of calculating a metabolite standard deviation value by performing linear regression on the 2SD spectrum, and a calculation of normalizing each of the metabolite content value and the metabolite standard deviation value.

BRIEF DESCRIPTION OF THE DRAWINGS

The above and other aspects of the present disclosure will become more apparent to those skilled in the art from a detailed description of exemplary embodiments of the present disclosure with reference to the accompanying drawings:

FIG. 1 is a block diagram for describing an electronic device according to the present disclosure;
FIG. 2 is a block diagram of an artificial intelligence (AI) device according to an embodiment of the present disclosure;
FIG. 3A illustrates an example of a pipeline of a quantification apparatus applicable to the present disclosure;
FIG. 3B illustrates examples of a simplified design of a Bayesian convolutional neural network that may be applied to the quantification apparatus of FIG. 3A and a workflow for training an artificial neural network;
FIG. 4 is an exemplary diagram of an operation of an input unit to which the present disclosure may be applied;
FIG. 5 is an exemplary diagram of an operation of a prediction unit to which the present disclosure may be applied;
FIG. 6 is an exemplary diagram of an operation of a post-processing unit to which the present disclosure may be applied;
FIG. 7 is exemplary diagram of normal operation verification to which the present disclosure may be applied; and
FIG. 8 is a flowchart illustrating an embodiment of an operation of a quantification apparatus to which the present disclosure may be applied.

The accompanying drawings, which are included as part of the detailed description to assist understanding of the present disclosure, illustrate embodiments of the present disclosure and explain the technical features of the present disclosure together with the detailed description.

DETAILED DESCRIPTION OF EXEMPLARY EMBODIMENTS

Hereafter, embodiments of the present disclosure will be described in detail with reference to the accompanying drawings and the same or similar components are given the same reference numerals regardless of the numbers of figures and are not repeatedly described. In addition, the terms "module" and "unit" for components used in the following description are used only to easily make the disclosure. Therefore, these terms do not have meanings or roles that distinguish from each other in themselves. Further, when it is determined that a detailed description for the known art related to the present disclosure may obscure the gist of the present disclosure, the detailed description will be omitted.
Terms including ordinal numbers such as "first," "second," etc., may be used to describe various components, but the components are not to be construed as being limited to the terms. The terms are used only to distinguish one component from another component.
It is to be understood that when a first element is referred to as being "connected to" or "coupled to" a second element, it may be connected directly to or coupled directly to the second element or be connected to or coupled to the second element with a third element interposed therebetween. On the other hand, it should be understood that when a first element is referred to as being "connected directly to" or "coupled directly to" a second element, it may be connected to or coupled to the second element without a third element interposed therebetween.
Singular forms are intended to include plural forms unless the context clearly indicates otherwise.
It will be further understood that terms "include" or "have" used in the present specification specify the presence of features, numerals, steps, operations, components, parts mentioned in the present specification, or combinations thereof, but do not preclude the presence or addition of one or more other features, numerals, steps, operations, components, parts, or combinations thereof.

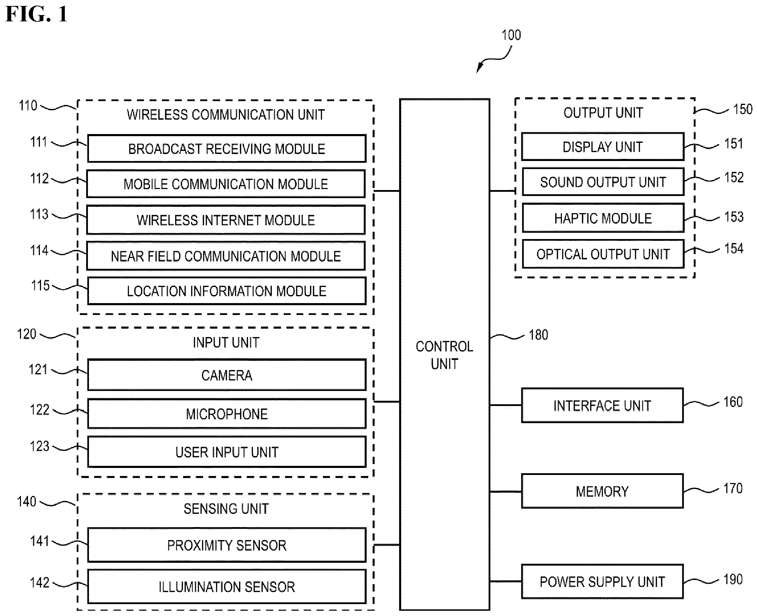

FIG. 1 is a block diagram for describing an electronic device according to the present disclosure.
An electronic device 100 may include a wireless communication unit 110, an input unit 120, a sensing unit 140, an output unit 150, an interface unit 160, a memory 170, a control unit 180, a power supply unit 190, and the like. The components illustrated in FIG. 1 are not essential to implementing the electronic device 100, so the electronic devices described herein may have more or fewer components than those listed above.
More specifically, the wireless communication unit 110 of the components may include one or more modules which enable wireless communication between the electronic device 100 and a wireless communication system, between the electronic device 100 and other electronic devices 100, or the electronic device 100 and an external server. In addition, the wireless communication unit 110 may include one or more modules which connect the electronic device 100 to one or more networks.
The wireless communication unit 110 may include at least one of a broadcast receiving module 111, a mobile communication module 112, a wireless Internet module 113, a near field communication module 114, and a location information module 115.
The input unit 120 may include a camera 121 or an image input unit for inputting an image signal, a microphone 122 or an audio input unit for inputting a sound signal, and a user input unit 123 for receiving information from a user. Examples of the user input unit 123 may include a touch key, a push key (mechanical key), and the like. Voice data or image data collected by the input unit 120 may be analyzed and processed as a control command of the user.

The sensing unit 140 may include one or more sensors for sensing at least one of information in the electronic device 100, information on an environment surrounding the electronic device 100, and user information. For example, the sensing unit 140 may include at least one of a proximity sensor 141, an illumination sensor 142, a touch sensor, an acceleration sensor, a magnetic sensor, a G-sensor, a gyroscope sensor, a motion sensor, an red-green-blue (RGB) sensor, an infrared sensor (IR sensor), a fingerprint sensor, an ultrasonic sensor, an optical sensor, an environmental sensor (e.g., a barometer, a hygrometer, a thermometer, a radiation detection sensor, a thermal detection sensor, a gas detection sensor, etc.), and a chemical sensor (e.g., an electronic nose sensor, a healthcare sensor, a biometric sensor, etc.). Meanwhile, the electronic device 100 may use a combination of information sensed by at least two or more of these sensors.

The output unit 150 is for generating an output related to a sight, hearing, or tactile sense. The output unit 150 may include at least one of a display unit 151, a sound output unit 152, a haptic module 153, and an optical output unit 154. The display unit 151 forms a layer structure with or is integrally formed with the touch sensor, thereby implementing a touch screen. The touch screen may function as the user input unit 123 which provides an input interface between the electronic device 100 and the user, and may provide an output interface between the electronic device 100 and the user.

The interface unit 160 serves as a path of various types of external devices connected to the electronic device 100. The interface unit 160 may include at least one of a wired/wireless headset port, an external charger port, a wired/wireless data port, a memory card port, a port for connection of devices including an identity module, an audio input/output (I/O) port, a video I/O port, and an earphone port. In the electronic device 100, an appropriate control related to the connected external device may be performed in response to the connection of the external device to the interface unit 160.

In addition, the memory 170 stores data supporting various functions of the electronic device 100. The memory 170 may store a plurality of application programs or applications that are driven by the electronic device 100, and data and instructions for operating the electronic device 100. At least some of these application programs may be downloaded from the external server via the wireless communication. In addition, at least some of these application programs may exist in the electronic device 100 at the time of shipment for basic functions (for example, an incoming and outgoing call function, and a message reception and transmission function) of the electronic device 100. Meanwhile, the application program may be stored in the memory 170, installed on the electronic device 100, and driven by the control unit 180 to perform the operation or function of the electronic device.

In addition to the operation related to the application program, the control unit 180 typically controls the overall operation of the electronic device 100. The control unit 180 may provide or process appropriate information or a function to a user by processing signals, data, information, and the like, which are input or output through the above-described components, or by driving an application program stored in the memory 170.

In addition, the control unit 180 may control at least some of the components described with reference to FIG. 1 to drive the application program stored in the memory 170. In addition, the control unit 180 may operate a combination of at least two or more of the components included in the electronic device 100 to drive the application program.

The power supply unit 190 receives power from an external power source and/or an internal power source under the control of the control unit 180 and supply the received power to each component included in the electronic device 100. The power supply unit 190 includes a battery which may be a built-in battery or a replaceable battery.

At least some of the components may operate in cooperation with each other in order to implement an operation, a control, or a control method of the electronic device 100 according to various embodiments described below. In addition, the operation, control, or control method of the electronic device 100 may be implemented on the electronic device 100 by driving at least one application program stored in the memory 170.

In this specification, the electronic device 100 may include an apparatus for quantifying metabolites in the body (hereinafter referred to as "quantification apparatus").

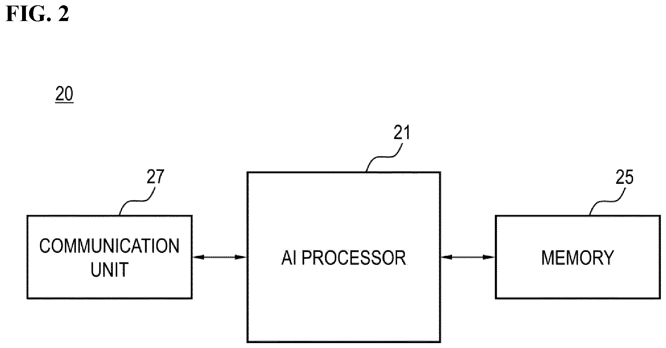

FIG. 2 is a block diagram of an artificial intelligence (AI) device according to an embodiment of the present disclosure.

The AI device 20 may include an electronic device including an AI module capable of performing AI processing, a server including the AI module, or the like. In addition, the AI device 20 may be included as at least a part of the electronic device 100 illustrated in FIG. 1 and may be provided to perform at least a part of the AI processing together.

Specifically, as illustrated in FIG. 2, the AI device 20 may include an AI processor 21, a memory 25, and/or a communication unit 27.

The AI device 20 is a computing device capable of learning neural networks, and may be implemented as various electronic devices such as a server, a desktop PC, a notebook PC, and a tablet PC.

The AI processor 21 may learn an artificial neural network using a program stored in the memory 25. In particular, the AI processor 21 may receive a one-dimensional MRS spectrum, predict a spectrum having improved signal quality compared to the received spectrum, and output the predicted spectrum. Here, examples of methods for improving signal quality may include denoising, frequency/phase-adjustment, and line-narrowing.

In addition, the AI processor 21 may form an artificial neural network mounted in a prediction unit to be described below.

Meanwhile, the AI processor 21 performing the functions as described above may be a general purpose processor (for example, central processing unit (CPU)), or may be an AI dedicated processor (for example, graphics processing unit (GPU)) for artificial intelligence learning.

The memory 25 may store various programs and data required for an operation of the AI device 20. The memory 25 may be implemented by a non-volatile memory, a volatile memory, a flash memory, a hard disc drive (HDD), a solid-state drive (SSD), or the like. The memory 25 is accessed by the AI processor 21, and readout/recording/correction/deletion/update, and the like of data in the memory 25 may be performed by the AI processor 21. In addition, the memory 25 may store a neural network model (e.g., a deep learning model) generated through a learning algorithm for data classification/recognition according to an embodiment of the present disclosure.

Meanwhile, the AI processor 21 may include a data learning unit which learns a neural network for data classification/recognition. For example, the data learning unit may acquire learning data to be used for learning, and apply the acquired learning data to the deep learning model, thereby training the deep learning model.

The communication unit 27 may transmit the AI processing result by the AI processor 21 to an external electronic device.

Here, the external electronic device may include other terminals and/or servers.

Meanwhile, the AI device 20 illustrated in FIG. 2 has been described functionally divided into the AI processor 21, the memory 25, the communication unit 27, and the like. However, the above-described components are integrated into one module, which may be referred to as an AI module or AI model.

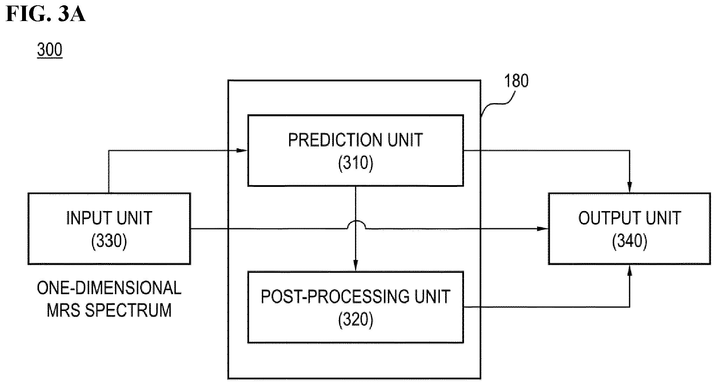

FIG. 3A illustrates a pipeline of a quantification apparatus applicable to the present disclosure.

Referring to FIG. 3A, a quantification apparatus 300 may include an input unit 330, a control unit 180, and an output unit 340, and the control unit 180 of the quantification apparatus 300 may include a prediction unit 310 and a post-processing unit 320.

In general, quantification of metabolites in MRS data was processed by a digital signal processing method based on a nonlinear-least-squares (NLLS) method, which causes problems with incomplete quantification results according to initial parameter conditions and algorithms.

As an uncertainty confidence index for the quantification results, a Cramér-Rao lower bound (CRLB) was used. Specifically, the related art empirically sets a CRLB criterion (20%, 30%, or 50%), and then determines that quantification results indicating a CRLB value higher than the criterion are "unreliable quantification results," and as a result, the "unreliable quantification results" are excluded without being used for research/diagnostic purposes.

However, since the statistical meaning of the CRLB is precision not accuracy, it has been reported that there is a risk of medical statistical bias when data screening is performed for the purpose of determining whether to use the quantification results based on the upper limit.

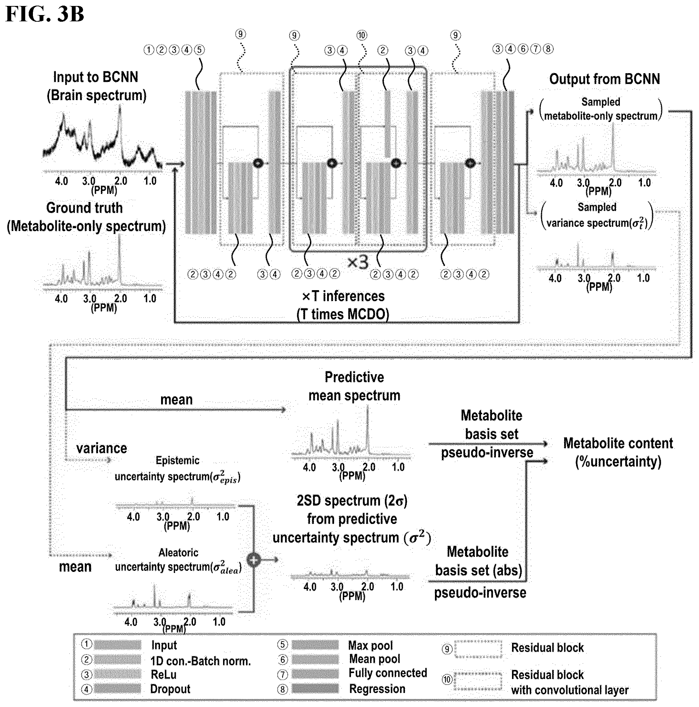

Therefore, the development of a metabolite quantification method, performance improvement, and a more accurate quantification result confidence index is very important to use MRS data for medical diagnosis purposes. In the present disclosure, the quantification apparatus 300 may provide improved metabolites quantification accuracy and uncertainty information. To this end, the prediction unit 310 may include an artificial neural network. The artificial neural network may be a Bayesian convolutional neural network (BCNN). FIG. 3B illustrates a simplified design of the BCNN and a workflow for training of the corresponding artificial neural network to estimate a metabolite content and associated uncertainty.

Referring to FIG. 3B, the input of the artificial neural network is a simulated human brain spectrum at 3.0T. It is typically degraded due to a low signal-to-noise ratio (SNR), line-broading, an MM signal, an unknown baseline, and phase/frequency shift. A ground truth target spectrum is a metabolite-only spectrum prior to adjusting a SNR, a linewidth, and a phase/frequency, and adding the MM signal. The output of the artificial neural network includes the metabolite-only spectrum and a noise variance spectrum. With T-time Monte Carlo dropout sampling, a total of T outputs per input spectrum is obtained (T=50). An average (predictive mean spectrum) of the metabolite-only output spectra is designated as a BCNN-predicted spectrum. The variance of the metabolite-only output spectra is designated as an epistemic (model) uncertainty spectrum. An average of the noise variance spectra is designated as an aleatoric (data) uncertainty spectrum. A two-standard deviation spectrum (2SD spectrum) is defined as a $2 \times$ standard deviation spectrum. A standard deviation spectrum (SD-spectrum) is acquired from (total uncertainty spectrum)$^{1/2}$. The total uncertainty spectrum is a sum of the aleatoric uncertainty spectrum and the epistemic uncertainty spectrum. Multiple regression is performed on both the BCNN-predicted spectrum and the 2SD spectrum using a metabolite basis set to estimate an individual metabolite content and associated uncertainty, respectively. The uncertainty is estimated from the 2SD spectrum. When the 2SD spectrum is given in an absolute mode, in this case, the metabolite basis set is also used as the absolute mode. Finally, the BCNN-predicted metabolite content is represented as "metabolite content (% uncertainty)." Here, the "% uncertainty" may be obtained by converting the estimated uncertainty into a percentage of each metabolite content.

Referring back to FIG. 3A, the prediction unit 310 may receive the one-dimensional MRS spectrum from the input unit 330 to predict a spectrum with improved signal quality of the corresponding spectrum, and may transmit first data, which is the predicted spectrum, to the output unit 340 and output the first data. The first data may correspond to the metabolite-only output spectra in the description referring to FIG. 3B.

In addition, the prediction unit 310 may transmit second data, which is a variance spectrum of an output spectrum predicted based on unsupervised learning, to the output unit 340 and output the first data and second data together. The second data may correspond to the noise variance spectra in the description referring to FIG. 3B.

The post-processing unit 320 may perform one-dimensional signal processing on each of the first data and the second data from the prediction unit 310, and then calculate third data, which is the metabolite content, and fourth data, which is a percentage standard deviation value of the metabolite.

The output unit 340 may receive the third data, the fourth data, and spectra (e.g., first data, second data, and pre-processed data of the input unit 330, etc.) of an intermediate stage that may be generated in the above-described pipeline and display the received third data, fourth data, and spectra on a user's screen.

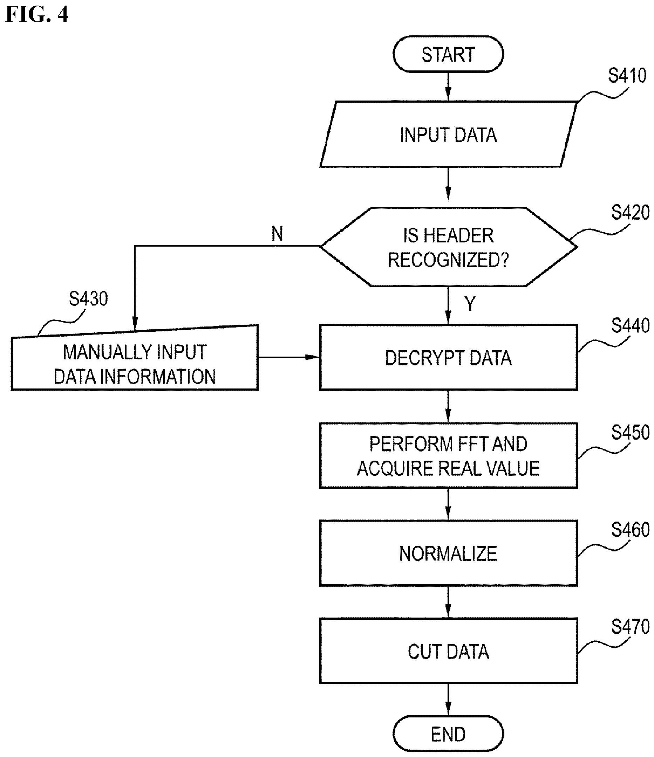

FIG. 4 is an exemplary diagram of an operation of an input unit to which the present disclosure may be applied.

Referring to FIG. 4, the input unit 330 may process input data and transmit the processed input data to the prediction unit 310 or the output unit 340.

The input unit 330 receives encrypted MRS data (S410).

Then, the input unit 330 determines whether a header of the encrypted MRS data can be recognized (S420).

As a result of the determination, when the header cannot be recognized (N, S420), a user may directly input MRS data information (S430).

As a result of the determination, when the header can be recognized (Y, S420), the input unit 330 may acquire a manufacturer and encryption information of the MRS data from the header.

Thereafter, the input unit 330 decrypts the MRS data based on the encryption information (S440).

The input unit 330 performs a fast Fourier transform (FFT) on the decrypted data and acquires a real value (S450).

The input unit 330 normalizes data of the real value (S460). Examples of the normalization methods may include zero to one, zero centered, interquartile range (IQR), and the like.

The input unit 330 indexes the normalized data and cuts the data so that the normalized data is to be input to the prediction unit 310 (S470).

The cut data, that is, the pre-processed data, may be transmitted to the prediction unit 310, vectorized in the prediction unit 310, and then displayed in the form of a spectrum picture in the output unit 340.

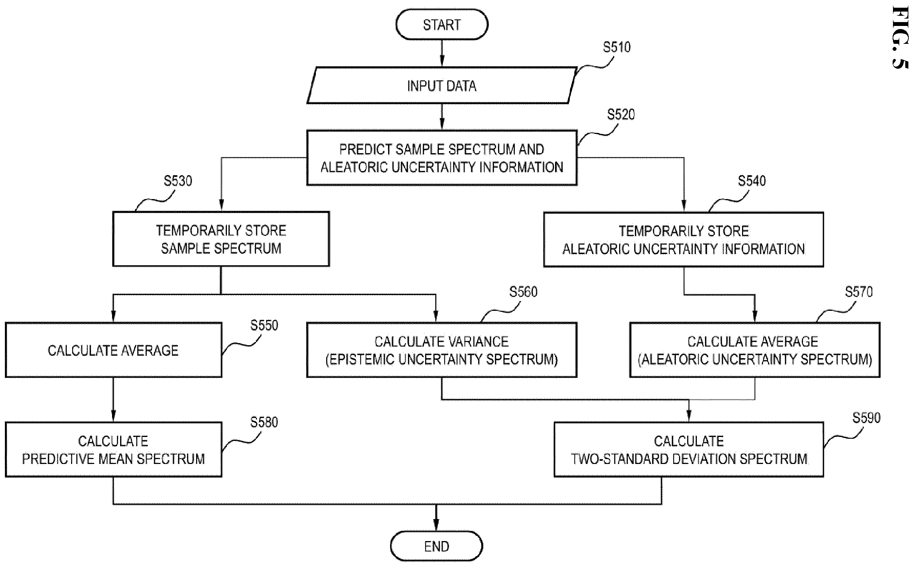

FIG. 5 is an exemplary diagram of an operation of a prediction unit to which the present disclosure may be applied.

Referring to FIG. 5, the prediction unit 310 receives the pre-processed data from the input unit 330 (S510).

The prediction unit 310 predicts a sample spectrum and aleatoric uncertainty information of the sample spectrum based on the pre-processed data (S520). For example, the prediction unit 310 may predict the sample spectrum and the aleatoric uncertainty information using an artificial neural network. In more detail, the artificial neural network loaded in the prediction unit 310 represents a variational inference network structure based on Bayesian approximation with Monte-Carlo dropout. The artificial neural network may predict and output, based on the unsupervised learning, aleatoric uncertainty information of each sample predicted through supervised learning. Here, the "sample spectrum" and the "aleatoric uncertainty information" may correspond to the "metabolite-only output spectra" and the "noise variance spectra" in the description referring to FIG. 3B, respectively. Such an artificial neural network may be pre-trained using a loss function considering heteroscedasticity. Big data may be used for pre-training.

As an example, the prediction unit 310 may predict the aleatoric uncertainty information by repeating as many times as the number of samples according to the number N of samples included in the pre-processed data. As another example, the number of times of repeated predictions may be empirically set by a user.

For example, when the prediction is performed N times, the prediction unit 310 may generate N sample spectra corresponding to the input spectrum and N aleatoric uncertainty spectra (noise variance spectra) corresponding to each sample spectrum.

As described above, when the sample spectrum and the aleatoric uncertainty information (noise variance spectrum) are predicted from the pre-processed data, the prediction unit 310 temporarily stores each of the predicted sample spectrum and the predicted aleatoric uncertainty information (S530 and S540). The sample spectrum stored in operation S530 may represent a form in which signal quality is improved compared to the spectrum of the input pre-processing data. Here, the form of the improved signal quality may mean a form in which one or more of denoising, frequency/phase-adjustment, and line-narrowing has been performed.

After operation S530, the prediction unit 310 calculates an average of the sample spectra (S550).

After operation S530, the prediction unit 310 calculates a variance of the sample spectra (S560). For example, the prediction unit 310 may derive the epistemic uncertainty spectrum through a variance calculation for the sample spectra and store the derived epistemic uncertainty spectrum.

In more detail, the prediction unit 310 may calculate the variance of the sample spectra using the artificial neural network and derive the epistemic uncertainty spectrum from a result of the calculation.

Meanwhile, after operation S540, the prediction unit 310 calculates the average of the aleatoric uncertainty information (noise variance spectra) for each sample spectrum (S570). For example, the prediction unit 310 may derive the aleatoric uncertainty spectrum through the average calculation of the aleatoric uncertainty information (noise variance spectra) for the sample spectra and store the derived aleatoric uncertainty spectrum.

In more detail, the prediction unit 310 may also predict the noise variance spectrum for the corresponding sample spectrum based on the unsupervised learning of the artificial neural network along with the sample spectrum. Accordingly, the corresponding noise variance spectrum may also be formed as many as the number of samples.

For example, when 50 sample spectra are generated, the same number of 50 noise variance spectra may also be generated, and the prediction unit 310 may calculate the average of the 50 noise variance spectra to derive the aleatoric uncertainty spectrum.

Here, the average of the sample spectra predicted by the artificial neural network may be used to quantify metabolites. The variance of the sample spectra predicted by the artificial neural network may be used as the epistemic uncertainty spectrum. The average of the variance spectra predicted by the artificial neural network may be used as the aleatoric uncertainty spectrum. The total uncertainty spectrum, which is the sum of the aleatoric uncertainty spectrum and the epistemic uncertainty spectrum, may be used for the 2SD spectrum. The 2SD spectrum may be used to estimate the uncertainty of the metabolite content by multiple regression in combination with the metabolite basis set in the absolute mode. That is, the 2SD spectrum may be used for the quantification standard deviation of the metabolite.

Meanwhile, after operation S550, the prediction unit 310 derives the predictive mean spectrum based on the average of the calculated sample spectra (S580).

For example, the prediction unit 310 may calculate the average of N sample spectra to calculate the mean spectrum. In more detail, when 50 sample spectra each having 1024 data points are generated, the prediction unit 310 may calculate the average of the 50 sample spectra for each data point to derive the predictive mean spectrum.

Meanwhile, after operation S570, the prediction unit 310 derives and stores the 2SD spectrum based on the epistemic uncertainty spectrum and the aleatoric uncertainty spectrum (S590).

For example, in the 2SD spectrum, "SD spectrum" is acquired from (total uncertainty spectrum)$^{1/2}$. The total uncertainty spectrum may be defined as "aleatoric uncertainty spectrum + epistemic uncertainty spectrum." The aleatoric uncertainty may be classified into homoscedastic aleatoric uncertainty and heteroscedastic aleatoric uncertainty.

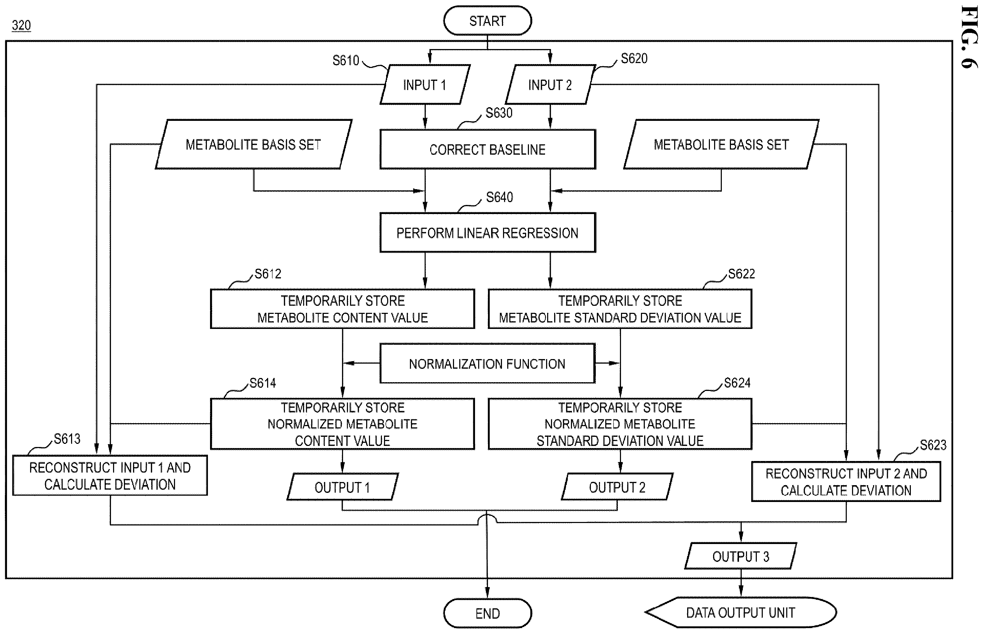

FIG. 6 is an exemplary diagram of an operation of a post-processing unit to which the present disclosure may be applied.

Referring to FIG. 6, the post-processing unit 320 receives the predictive mean spectrum from the prediction unit 310 (S610). In addition, the post-processing unit 320 receives the 2SD spectrum from the prediction unit 310 (S620). In FIG. 6, "input 1" and "input 2" mean the predictive mean spectrum and the 2SD spectrum, respectively.

The post-processing unit 320 performs baseline correction on each of the predictive mean spectrum and the 2SD spectrum (S630). For example, the post-processing unit 320 may correct all regions without a resonance spectrum to zero through the baseline correction.

The post-processing unit 320 performs linear regression on the baseline-corrected data using the metabolite basis set (S640).

For example, unlike in vivo MRS spectra in which signal quality is low and individual metabolite spectra are overlapped, the metabolite basis set includes a set of high-quality reference MRS spectra for each metabolite.

In addition, each metabolite spectrum constituting the metabolite basis set needs to be acquired under exactly the same conditions as the MRS scan parameters (e.g., repetition time (TR), echo time (TE), localization pulse sequence, etc.) when acquiring the in vivo MRS spectrum.

For example, the metabolite basis set, which may be used when quantifying 17 metabolites of the in vivo MRS spectrum having 1024 data points, may be constituted in a matrix form of 17×1024, and may be configured as a set of spectra in independent vector form for each metabolite.

Here, as a method of acquiring each metabolite spectrum constituting the metabolite basis set, the following may be exemplified.

1) Quantum mechanical calculation using a density matrix. That is, an approximated simulation method; Under specific MRS scan parameters (TR, TE, localization pulse sequence), a response shown by quantum mechanical properties (Number of spin, Chemical shift, J-coupling) in a magnetic field environment due to a molecular structure of each metabolite.

2) A method of acquiring data by preparing each metabolite sample as a high-concentration aqueous solution, putting the high-concentration aqueous solution in a spherical glass ball, etc., and performing the MRS scan with the same protocol as the targeted in vivo MRS spectrum.

The post-processing unit 320 calculates a metabolite content value from the predictive mean spectrum through the linear regression and temporarily stores the calculated metabolite content value (S612).

The post-processing unit 320 calculates a metabolite standard deviation value from the 2SD spectrum through linear regression and temporarily stores the calculated metabolite standard deviation value (S622).

For example, singular value decomposition (SVD) through a Moore-Penrose pseudo inverse matrix may be performed in the linear regression.

The post-processing unit 320 normalizes each of the metabolite content value and the metabolite standard deviation value through a normalization function, and temporarily stores each of the normalized metabolite content value and the normalized metabolite standard deviation value (S614 and S624).

For example, the normalization process may be calculated according to Equation 1. Specifically, considering a relative content of metabolite to water or a relative content to a reference metabolite (e.g., Creatine+Phosphocreatine), the relative content of metabolite to water may be calculated according to Equation 1 below.

[Equation 1]

$$Conc_{meta} = 55 * \left(\frac{S_{meta}}{S_{water}}\right) * CorrTiss * \frac{Nspins_{water}}{Nspins_{meta}}$$

In Equation 1, the $Conc_{meta}$ denotes a content of a target metabolite (mmol/L). The $S_{meta}$ and the $S_{water}$ denote a signal area of the metabolite and a signal area of the water, respectively. The CorrTiss denotes a ratio of water in a biological tissue from which the MRS signal is obtained. The $Nspins_{water}$ denotes the number of spins of water participating in generating a resonance signal, and the $Nspins_{meta}$ denotes the number of spins of the target metabolite participating in generating the resonance signal. Equation 1 may be modified by adding a correction term considering a T1 relaxation time and a T2 relaxation time of water, and the T1 relaxation time and T2 relaxation time of the metabolite. The post-processing unit 320 may convert each of the normalized metabolite content value and the normalized metabolite standard deviation value into a vector form and transmit the converted normalized metabolite content value and normalized metabolite standard deviation value to the output unit 340.

In addition, the post-processing unit 320 may take scalar products of resonance signals of metabolites in the metabolite basis set corresponding to the metabolite content values and metabolite content values, sum all the scalar products to form a reconstructed signal, and obtain a signal representing a difference between an input value (input 1) and the reconstructed signal (S613).

In addition, the post-processing unit 320 may take scalar products of resonance signals of metabolites in the metabolite basis set corresponding to the normalized metabolite standard deviation values and the normalized metabolite standard deviation values, sum all the scalar products to form a reconstructed signal, and obtain a signal representing a difference between an input value (input 2) and the reconstructed signal (S623). For example, the metabolite basis set used in such a deviation-related calculation may have a form converted into an absolute value form.

The reconstructed signal may be used to check whether the normalized metabolite content value and the normalized metabolite standard deviation value have actually been calculated correctly.

For example, by using the MRS spectrum having 1024 data points, both the predicted mean spectrum and the 2SD spectrum may each represent 1024 data points.

In this case, the linear regression (pinv) process based on the Moore-Penrose pseudo inverse matrix for 17 kinds of "metabolite content calculations" having 1024 data points includes "metabolite content calculation process and metabolite standard deviation calculation process," and the calculation equation for each process is as follows. In the calculation equation below, pinv means a pseudo-inverse matrix, and abs means an absolute value.

- metabolite context [1 × 17 vector] = predictive mean spectrum [1 × 1024 vector] × pinv (metabolite basis set) [1024 × 17 matrix] - metabolite strandard deviation [17] = standard deviation spectrum [1024] × pinv (abs(metabolite basis set)) [1024 × 17]

Meanwhile, the reconstruction includes a reconstructed spectrum calculation process for the predictive mean spectrum and a reconstructed spectrum calculation process for a 2×standard deviation spectrum, and the calculation equation for each process is as follows. In the calculation equation below, sum means the summation, and abs means the absolute value.

- Reconstructed spectrum of predictive mean spectrum [1024] = sum (metabolite content × (metabolite basis set)) - Reconstructed spectrum of 2SD spectrum [1024] = sum (metabolite standard deviation × (abs(metabolite basis set)))

The post-processing unit 320 may check a difference between the reconstructed spectra and each reference spectrum. Based on the check result, it may be checked whether the prediction unit 310 normally operates.

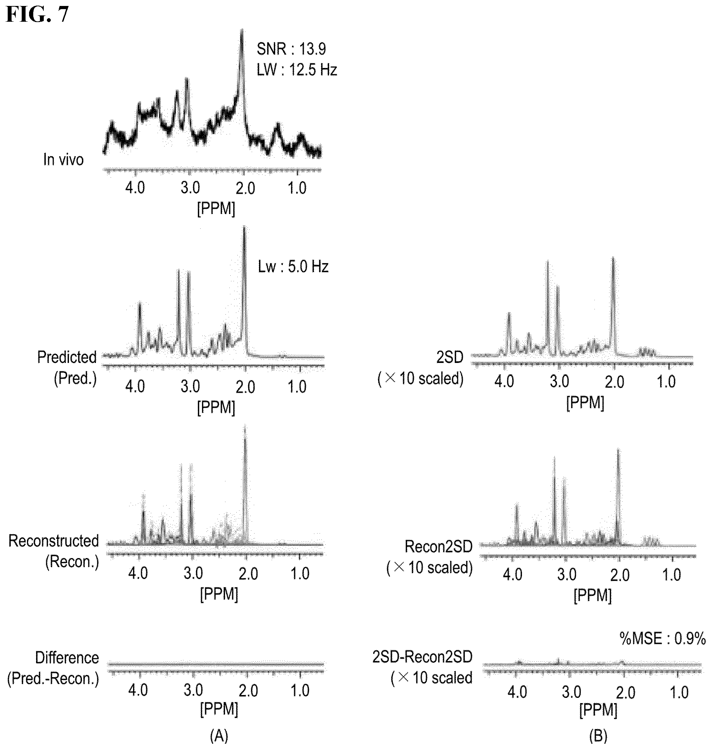

FIG. 7 is exemplary diagram of normal operation verification to which the present disclosure may be applied.

FIG. 7(A) illustrates, in order from the top, a target in vivo brain MRS spectrum (in vivo), a predictive mean spectrum (Pred.), a reconstructed predictive mean spectrum (Recon.), and a difference (Pred.-Recon.) between the predictive mean spectrum and the reconstructed predictive mean spectrum. FIG. 7(B) illustrates, in order from the top, a 2×standard deviation spectrum (2SD), a reconstructed 2×standard deviation spectrum (Recon2SD), and a difference (2SD-Recon2SD) between a 2×standard deviation spectrum (2SD) and a reconstructed 2×standard deviation spectrum (Recon2SD).

Referring back to FIG. 6, the post-processing unit 320 may convert the spectra acquired in operations S613 and S623, into a vector matrix, and then transmit the converted vector matrix to the data output unit included in the output unit 340.

The output unit 340 may receive and output the following data.

- From the input unit 330, pre-processed MRS data
- From the prediction unit 310, predictive mean spectrum and 2SD spectrum (2SD spectrum)
- From the post-processing unit 320, normalized metabolite content value (metabolite normalization value), normalized metabolite standard deviation value (metabolite standard deviation normalization value), difference spectrum between normalized metabolite content value and predictive mean spectrum (S610), and difference spectrum between normalized metabolite standard deviation and SD spectrum (S620).

The output unit 340 may display each input as a result on the user's screen.

For example, a series of quantification processes may be arranged and displayed in order, from the MRS data acquired from a patient and pre-processed to the quantification result spectrum (artificial neural network prediction spectrum and reconstructed spectrum), and in addition to this, quantification content for each metabolite and confidence index information may be displayed together.

In addition, the interface of the output unit 340 may be configured as a user-responsive type capable of displaying individual quantification results of each metabolites desired by the user. The interface may also include an option to output the quantification results to the outside of the device in the form of a spreadsheet or image.

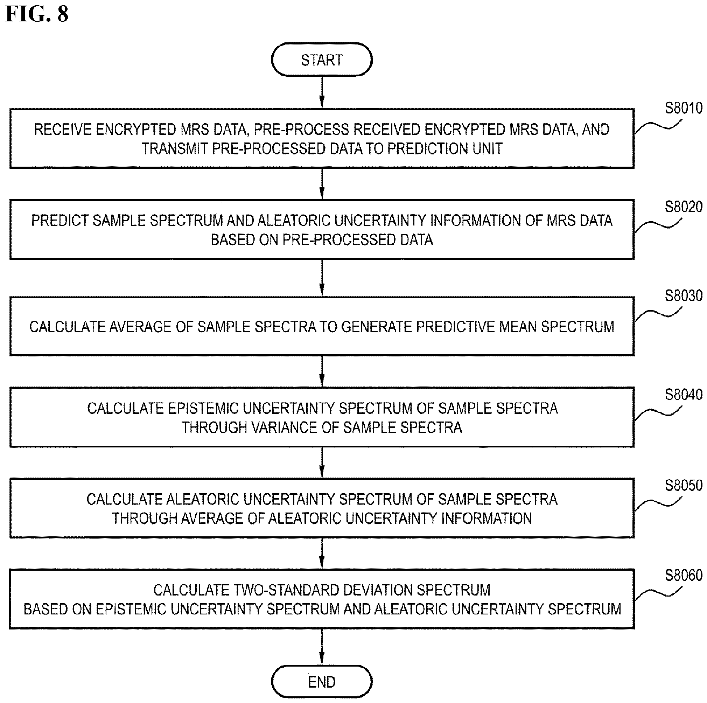

FIG. 8 is a flowchart illustrating an embodiment of an operation of a quantification apparatus to which the present disclosure may be applied.

Referring to FIG. 8, the quantification apparatus 300 may include the input unit 330, the prediction unit 310, the post-processing unit 320, and the output unit 340 described above.

The quantification apparatus 300 receives encrypted MR spectroscopy (MRS) data through the input unit 330, pre-processes the received encrypted MRS data, and transmits the pre-processed data to the prediction unit 310 (S8010). For example, the pre-processing may include a decrypting operation, an operation of acquiring a real value through fast Fourier transform, an operation of normalizing a real value, an indexing operation, and an operation of cutting data to be input to the prediction unit 310.

The quantification apparatus 300 predicts the sample spectrum and aleatoric uncertainty information (noise dispersion spectrum) of the MRS data based on the pre-processed data through the artificial neural network included in the prediction unit 310 (S8020). For example, the artificial neural network may be the Bayesian convolutional Neural Network (BCNN), and the prediction unit 310 may predict the aleatoric uncertainty information (noise dispersion spectrum) through the artificial neural network. When the number of samples included in the pre-processed data is plural, the prediction unit 310 may repeatedly predict the aleatoric uncertainty information (noise dispersion spectrum) according to the number of samples.

In addition, the sample spectra may have the form in which signal quality is improved compared to the spectrum of the pre-processed data.

The quantification apparatus 300 calculates the average of the sample spectra to generate the predictive mean spectrum (S8030). For example, the predictive mean spectrum may be generated based on an average value of sample spectra for each data point.

The quantification apparatus 300 calculates the epistemic uncertainty spectrum of sample spectra (S8040).

For example, the prediction unit 310 may calculate the epistemic uncertainty spectrum through the variance calculation for the sample spectra.

The quantification apparatus 300 calculates the aleatoric uncertainty spectrum of sample spectra (S8050). For example, the prediction unit 310 may calculate the aleatoric uncertainty spectrum of the sample spectra through the average of the aleatoric uncertainty information.

The quantification apparatus 300 calculates the 2SD spectrum of the sample spectra based on the epistemic uncertainty spectrum and the aleatoric uncertainty spectrum (S8060).

The quantification apparatus 300 transmits the predictive mean spectrum and the 2SD spectrum to the post-processing unit 320 and the output unit 340. For example, the post-processing unit 320 may determine whether the quantification of quantification apparatus 300 is normally operating.

As a result, the present specification relates to the method of quantifying metabolites in the body through MRS data, and can provide metabolites-based medical information with significantly improved quantification accuracy compared to the existing technologies.

In addition, the reliability information of the quantification results shows significantly improved performance compared to the existing methods, enabling screening for uncertain quantification results, and can quantity metabolites at a much faster speed than the existing methods, and as a result, can be used for precise quantification of metabolites and for diagnosis assistance immediately after MRS scan.

The present specification described above can be embodied as a computer readable code on a medium in which a program is recorded. A computer readable medium may include all kinds of recording devices in which data that may be read by a computer system are stored. An example of the computer readable medium may include a hard disk drive (HDD), a solid state disk (SSD), a silicon disk drive (SDD), a read only memory (ROM), a random access memory (RAM), a compact disk read only memory (CD-ROM), a magnetic tape, a floppy disk, an optical data storage, and the like, and also include a medium implemented in a form of a carrier wave (for example, transmission through the Internet). Therefore, the above-described detailed description is to be interpreted as being illustrative rather than being restrictive in all aspects.

According to an embodiment of the present disclosure, it is possible to improve the accuracy of quantification of metabolites and the uncertainty information of quantification of metabolites by constructing a Bayesian convolutional neural network (BCNN)-based variational inference network optimized for an MRS data format and developing a pre-processing algorithm and post-processing algorithm of the network.

Effects which can be achieved by the present disclosure are not limited to the above-described effects. That is, other effects that are not described may be obviously understood by those skilled in the art to which the present disclosure pertains from the above detailed description.

In addition, although the services and embodiments have been mainly described hereinabove, this is only an example and does not limit the present specification.

**Claims**

1. A computer-implemented method for non-invasive quantification of metabolites in a body, comprising:

   a) predicting (S520; S8020) aleatoric uncertainty information and a sample spectrum from a pre-processed input spectrum using an artificial neural network;
   b) generating (S8030) a predictive mean spectrum by calculating an average of the sample spectrum;
   c) calculating (S560, S8040) an epistemic uncertainty spectrum of the sample spectrum based on a variance of the sample spectrum;
   d) calculating (S570, S8050) an aleatoric uncertainty spectrum of the sample spectrum based on an average of the aleatoric uncertainty information;
   e) calculating (S590, S8060) a two-standard deviation, 2SD, spectrum of the sample spectrum based on the epistemic uncertainty spectrum and the aleatoric uncertainty spectrum;
   f1) performing baseline correction on the predictive mean spectrum and the 2SD spectrum;
   f2) calculating a metabolite content value by performing linear regression on the corrected predictive mean spectrum;
   f3) calculating a metabolite standard deviation value by performing linear regression on the corrected 2SD spectrum; and
   f4) normalizing the metabolite content value and the metabolite standard deviation value;
   g) wherein the normalizing is based on (1) a relative content of the metabolite to water or (2) a relative content of the metabolite to a reference metabolite;
   h) wherein the relative content of the metabolite to the water is calculated based on Equation 1,

[Equation 1]

$$Conc_{meta} = 55 * \left(\frac{S_{meta}}{S_{water}}\right) * CorrTiss * \frac{Nspins_{water}}{Nspins_{meta}},$$

where the $Conc_{meta}$ denotes a content of a target metabolite (mmol/L),

the $S_{meta}$ denotes a signal area of the target metabolite,
the $S_{water}$ denotes a signal area of the water,
the CorrTiss denotes a ratio of water in a biological tissue from which a magnetic resonance spectroscopy (MRS) signal is obtained,
the $Nspins_{water}$ denotes the number of spins of water participating in generating a resonance signal, and
the $Nspins_{meta}$ denotes the number of spins of the target metabolite participating in generating the resonance signal.

2. The method of claim 1, wherein the artificial neural network includes a Bayesian convolutional neural network (BCNN).

3. The method of claim 1 or 2, wherein, when compared to the pre-processed input spectrum, a signal-to-noise ratio (SNR), a linewidth, and a phase/frequency are adjusted in the sample spectrum.

4. The method of claim 1, wherein the predicting includes predicting T sample spectra and T pieces of aleatoric uncertainty information per the pre-processed input spectrum through T times Monte Carlo dropout sampling (MCDO sampling),

the sample spectrum corresponds to a metabolite-only output spectrum, and
the aleatoric uncertainty information corresponds to a noise variance spectrum.

5. The method of claim 4, wherein the predictive mean spectrum is generated based on an average value of the sample spectra for each data point.

6. The method of claim 1, wherein the calculating of the 2SD spectrum includes:

calculating a total uncertainty spectrum by summing the epistemic uncertainty spectrum and the aleatoric uncertainty spectrum;
calculating a standard deviation spectrum (SD-spectrum) from a 1/2 power of the total uncertainty spectrum; and
calculating the 2SD spectrum by multiplying the SD spectrum by 2.

7. The method of claim 1, further comprising
converting the normalized metabolite content value and the normalized metabolite standard deviation value into a vector form and transmitting the converted vector to an output unit (150, 152, 154, 340).

8. The method of claims 1 or 7, further comprising:

forming a reconstructed signal by calculating a difference between the normalized metabolite content value and the predictive mean spectrum;
forming a reconstructed signal by calculating a difference between the normalized metabolite standard deviation value and the 2SD spectrum; and
checking accuracy of the normalization based on a result of comparing the reconstructed signals.

9. The method of any one of claims 1 to 8, further comprising transmitting the predictive mean spectrum and the 2SD spectrum to an output unit (150, 152, 154, 340).

10. A quantification apparatus (300) for quantifying metabolites in a body, comprising:

one or more processors (21); and
one or more memories (25) configured to store instructions that, when executed by the one or more processors, cause the one or more processors to perform a calculation,
wherein the calculation performed by the one or more processors (21) includes:

a) a calculation of pre-processing (S410-S470) encrypted magnetic resonance spectroscopy (MRS) data to provide an input spectrum;

b) a calculation of predicting (S520) aleatoric uncertainty information and a sample spectrum from the pre-processed input spectrum using an artificial neural network;

c) a calculation of calculating (S580, S590) a predictive mean spectrum of the predicted sample spectrum and a two-standard deviation, 2SD, spectrum of the predicted sample spectrum based on the predicted sample spectrum and the predicted aleatoric uncertainty information;

d) a calculation of performing baseline correction on the predictive mean spectrum and the 2SD spectrum;

e) a calculation of calculating (S612) a metabolite content value by performing (S640) linear regression on the corrected predictive mean spectrum;

f) a calculation of calculating (S622) a metabolite standard deviation value by performing (S640) linear regression on the corrected 2SD spectrum; and

g) a calculation of normalizing (S614, S624) each of the metabolite content value and the metabolite standard deviation value;

h) wherein the normalizing calculation is based on (1) a relative content of the metabolite to water or (2) a relative content of the metabolite to a reference metabolite;

i) wherein the relative content of the metabolite to the water is calculated based on Equation 1,

[Equation 1]
$$Conc_{meta} = 55 * \left(\frac{S_{meta}}{S_{water}}\right) * CorrTiss * \frac{Nspins_{water}}{Nspins_{meta}},$$

where the $Conc_{meta}$ denotes a content of a target metabolite (mmol/L),

the $S_{meta}$ denotes a signal area of the target metabolite,

the $S_{water}$ denotes a signal area of the water,

the CorrTiss denotes a ratio of water in a biological tissue from which a magnetic resonance spectroscopy (MRS) signal is obtained,

the $Nspins_{water}$ denotes the number of spins of water participating in generating a resonance signal, and

the $Nspins_{meta}$ denotes the number of spins of the target metabolite participating in generating the resonance signal.

**11.** A non-transitory computer-readable storage medium for storing a computer program for executing the quantification method of any one of claims 1 to 9.

## Patentansprüche

**1.** Computerimplementiertes Verfahren zur nicht-invasiven Quantifizierung von Metaboliten in einem Körper, umfassend:

a) Vorhersagen (S520; S8020) von aleotorischer Unsicherheitsinformation und eines Probenspektrums aus einem vorverarbeiteten Eingangsspektrum unter Verwendung eines künstlichen neuronalen Netzwerks;

b) Erzeugen (S8030) eines prädiktiven Durchschnittsspektrums durch Berechnen eines Durchschnitts des Probenspektrums;

c) Berechnen (S560, S8040) eines epistemischen Unsicherheitsspektrums des Probenspektrums basierend auf einer Varianz des Probenspektrums;

d) Berechnen (S570, S8050) eines aleatorischen Unsicherheitsspektrums des Probenspektrums basierend auf einem Durchschnitt der aleatorischen Unsicherheitsinformation;

e) Berechnen (S590, S8060) eines Zwei-Standard-Abweichungs-, 2SD-, Spektrums des Probenspektrums basierend auf dem epistemischen Unsicherheitsspektrum und dem aleatorischen Unsicherheitsspektrum;

f1) Durchführen einer Basislinienkorrektur an dem prädiktiven Durchschnittsspektrum und dem 2SD-Spektrum;

f2) Berechnen eines Metabolitgehaltwerts durch Durchführen einer linearen Regression an dem korrigierten prädiktiven Durchschnittsspektrum;

f3) Berechnen eines Metabolitstandardabweichungswerts durch Durchführen einer linearen Regression an dem korrigierten 2SD-Spektrum; und

f4) Normalisieren des Metabolitgehaltwerts und des Metabolitstandardabweichungswerts;

g) wobei das Normalisieren auf (1) einem relativen Gehalt des Metaboliten zu Wasser oder (2) einem relativen Gehalt des Metaboliten zu einem Referenzmetaboliten basiert;

h) wobei der relative Gehalt des Metaboliten zu Wasser basierend auf Gleichung 1 berechnet wird,

[Gleichung 1]

$$Conc_{meta} = 55 * \left(\frac{S_{meta}}{S_{water}}\right) * CorrTiss * \frac{Nspins_{water}}{Nspins_{meta}},$$

wobei das $Conc_{meta}$ einen Gehalt eines Zielmetaboliten (mmol/l) bezeichnet,

das $S_{meta}$ eine Signalfläche des Zielmetaboliten bezeichnet,
das $S_{water}$ eine Signalfläche des Wassers bezeichnet,
das CorrTiss ein Verhältnis von Wasser in einem biologischen Gewebe, aus dem ein Magnetresonanzspektroskopie(MRS)-Signal erhalten wird, bezeichnet,
das $Nspins_{water}$ die Anzahl von Spins von Wasser, die an der Erzeugung eines Resonanzsignals beteiligt sind, bezeichnet, und
das $Nspins_{meta}$ die Anzahl von Spins des Zielmetaboliten, die an der Erzeugung des Resonanzsignals beteiligt sind, bezeichnet.

2. Verfahren nach Anspruch 1, wobei das künstliche neuronale Netzwerk ein Bayessches neuronales Faltungsnetzwerk (Bayesian Convolutional Neural Network, BCNN) umfasst.

3. Verfahren nach Anspruch 1 oder 2, wobei im Vergleich zu dem vorverarbeiteten Eingangsspektrum ein Signal-Rausch-Verhältnis (SNR), eine Linienbreite und eine Phase/Frequenz im Probenspektrum angepasst werden.

4. Verfahren nach Anspruch 1, wobei das Vorhersagen das Vorhersagen von T Probenspektren und T Stücken von aleatorischer Unsicherheitsinformation pro vorverarbeitetem Eingangsspektrum durch T-mal Monte-Carlo-Dropout-Sampling (MCDO-Sampling) umfasst,

das Probenspektrum einem Nur-Metaboliten-Ausgangsspektrum entspricht, und
die aleatorische Unsicherheitsinformation einem Rauschvarianzspektrum entspricht.

5. Verfahren nach Anspruch 4, wobei das prädiktive Durchschnittsspektrum basierend auf einem Durchschnittswert der Probenspektren für jeden Datenpunkt erzeugt wird.

6. Verfahren nach Anspruch 1, wobei das Berechnen des 2SD-Spektrums umfasst:

Berechnen eines Gesamtunsicherheitsspektrums durch Summieren des epistemischen Unsicherheitsspektrums und des aleatorischen Unsicherheitsspektrums;
Berechnen eines Standardabweichungsspektrums (SD-Spektrum) aus einer 1/2-Potenz des Gesamtunsicherheitsspektrums; und
Berechnen des 2SD-Spektrums durch Multiplizieren des SD-Spektrums mit 2.

7. Verfahren nach Anspruch 1, ferner umfassend
Umwandeln des normalisierten Metabolitgehaltwerts und des normalisierten Metabolitstandardabweichungswerts in eine Vektorform und Übertragen des umgewandelten Vektors an eine Ausgabeeinheit (150, 152, 154, 340).

8. Verfahren nach Anspruch 1 oder 7, ferner umfassend:

Bilden eines rekonstruierten Signals durch Berechnen einer Differenz zwischen dem normalisierten Metabolitgehaltwert und dem prädiktiven Durchschnittsspektrum;
Bilden eines rekonstruierten Signals durch Berechnen einer Differenz zwischen dem normalisierten Metabolitstandardabweichungswert und dem 2SD-Spektrum; und
Prüfen der Genauigkeit der Normalisierung basierend auf einem Ergebnis des Vergleichs der rekonstruierten Signale.

9. Verfahren nach einem der Ansprüche 1 bis 8, ferner umfassend ein Übertragen des prädiktiven Durchschnittsspektrums und des 2SD-Spektrums an eine Ausgabeeinheit (150, 152, 154, 340).

10. Quantifizierungsvorrichtung (300) zur Quantifizierung von Metaboliten in einem Körper, umfassend:

einen oder mehrere Prozessoren (21); und
einen oder mehrere Speicher (25), die konfiguriert sind, um Anweisungen zu speichern, die, wenn sie von dem einen oder den mehreren Prozessoren ausgeführt werden, den einen oder die mehreren Prozessoren veranlassen, eine Berechnung durchzuführen,
wobei die von dem einen oder den mehreren Prozessoren (21) durchgeführte Berechnung umfasst:

a) eine Berechnung zum Vorverarbeiten (S410-S470) von verschlüsselten Magnetresonanzspektroskopie(MRS)-Daten, um ein Eingangsspektrum bereitzustellen;
b) eine Berechnung zum Vorhersagen (S520) von aleotorischer Unsicherheitsinformation und eines Probenspektrums aus dem vorverarbeiteten Eingangsspektrum unter Verwendung eines künstlichen neuronalen Netzwerks;
c) eine Berechnung zum Berechnen (S580, S590) eines prädiktiven Durchschnittsspektrums des vorhergesagten Probenspektrums und eines Zwei-Standard-Abweichungs-, 2SD-, Spektrums des vorhergesagten Probenspektrums basierend auf dem vorhergesagten Probenspektrum und der vorhergesagten aleatorischen Unsicherheitsinformation;
d) eine Berechnung zum Durchführen einer Basislinienkorrektur an dem prädiktiven Durchschnittsspektrum und dem 2SD-Spektrum;
e) eine Berechnung zum Berechnen (S612) eines Metabolitgehaltwerts durch Durchführen (S640) einer linearen Regression an dem korrigierten prädiktiven Durchschnittsspektrum;
f) eine Berechnung zum Berechnen (S622) eines Metabolitstandardabweichungswerts durch Durchführen (S640) einer linearen Regression an dem korrigierten 2SD-Spektrum; und
g) eine Berechnung zum Normalisieren (S614, S624) jeweils des Metabolitgehaltwerts und des Metabolitstandardabweichungswerts;
h) wobei die Normalisierungsberechnung auf (1) einem relativen Gehalt des Metaboliten zu Wasser oder (2) einem relativen Gehalt des Metaboliten zu einem Referenzmetaboliten basiert;
i) wobei der relative Gehalt des Metaboliten zu Wasser basierend auf Gleichung 1 berechnet wird,

[Gleichung 1]

$$Conc_{meta} = 55 * \left(\frac{S_{meta}}{S_{water}}\right) * CorrTiss * \frac{Nspins_{water}}{Nspins_{meta}},$$

wobei das $Conc_{meta}$ einen Gehalt eines Zielmetaboliten (mmol/l) bezeichnet,

das $S_{meta}$ eine Signalfläche des Zielmetaboliten bezeichnet,
das $S_{water}$ eine Signalfläche des Wassers bezeichnet,
das CorrTiss ein Verhältnis von Wasser in einem biologischen Gewebe, aus dem ein Magnetresonanzspektroskopie(MRS)-Signal erhalten wird, bezeichnet,
das $Nspins_{water}$ die Anzahl von Spins von Wasser, die an der Erzeugung eines Resonanzsignals beteiligt sind, bezeichnet, und
das $Nspins_{meta}$ die Anzahl von Spins des Zielmetaboliten, die an der Erzeugung des Resonanzsignals beteiligt sind, bezeichnet.

11. Nicht-transitorisches computerlesbares Speichermedium zum Speichern eines Computerprogramms zum Ausführen des Quantifizierungsverfahrens nach einem der Ansprüche 1 bis 9.

**Revendications**

1. Procédé mis en oeuvre par un ordinateur pour la quantification non invasive de métabolites dans le corps, comprenant :

a) la prévision (S520 ; S8020) d'informations d'incertitude aléatoire et d'un spectre d'échantillon à partir d'un spectre d'entrée pré-traité au moyen d'un réseau neuronal artificiel ;

b) la génération (S8030) d'un spectre moyen prédictif par calcul d'une moyenne du spectre d'échantillon ;

c) le calcul (S560, S8040) d'un spectre d'incertitude épistémique du spectre d'échantillon sur la base d'un écart du spectre d'échantillon ;

d) le calcul (S570, S8050) d'un spectre d'incertitude aléatoire du spectre d'échantillon sur la base d'une moyenne des informations d'incertitude aléatoire ;

e) le calcul (S590, S8060) d'un spectre d'écart à deux normes, 2SD, du spectre d'échantillon sur la base du spectre d'incertitude épistémique et du spectre d'incertitude aléatoire ;

f1) l'exécution d'une correction de ligne de base sur le spectre moyen prédictif et le spectre 2SD ;

f2) le calcul d'une valeur de teneur en métabolites par exécution d'une régression linéaire sur le spectre moyen prédictif corrigé ;

f3) le calcul d'une valeur d'écart standard des métabolites par exécution d'une régression linéaire sur le spectre 2SD corrigé ; et

f4) la normalisation de la valeur de teneur en métabolites et la valeur d'écart standard des métabolites ;

g) où la normalisation est basée sur (1) une teneur relative des métabolites par rapport à l'eau ou (2) une teneur relative des métabolites par rapport à une métabolite de référence ;

h) où la teneur relative des métabolites par rapport à l'eau est calculée sur la base de l'équation 1,

[Equation 1]

$$Conc_{meta} = 55 * \left(\frac{S_{meta}}{S_{water}}\right) * CorrTiss * \frac{Nspins_{water}}{Nspins_{meta}},$$

où $Conc_{meta}$ désigne une teneur d'une métabolite cible (mmol/L),

$S_{meta}$ désigne une zone de signal de la métabolite cible,
$S_{water}$ désigne une zone de signal de l'eau,
CorrTiss désigne un rapport de l'eau dans un tissu biologique dont un signal de spectroscopie par résonance magnétique (MRS) est obtenu,
$Nspins_{water}$ désigne le nombre de spins d'eau participant à la génération d'un signal de résonance, et
$Nspins_{meta}$ désigne le nombre de spins de la métabolite cible participant à la génération du signal de résonance.

2. Procédé selon la revendication 1, où le réseau neuronal artificiel comprend un réseau neuronal convolutif bayésien (BCNN).

3. Procédé selon la revendication 1 ou la revendication 2, où, par comparaison avec le spectre d'entrée pré-traité, un rapport signal-bruit (SNR), une largeur de ligne, et une phase/fréquence sont réglés dans le spectre d'échantillon.

4. Procédé selon la revendication 1, où la prévision comprend la prévision de T spectres d'échantillon et de T éléments d'informations d'incertitude aléatoire pour chaque spectre d'entrée pré-traité par T échantillonnages de Monte-Carlo (échantillonnage MCDO),

le spectre d'échantillon correspond à un spectre de sorte de métabolites seulement, et
les informations d'incertitude aléatoire correspondent à un spectre de variance de bruit.

5. Procédé selon la revendication 4, où le spectre moyen prédictif est généré sur la base d'une valeur moyenne des spectres d'échantillon pour chaque point de données.

6. Procédé selon la revendication 1, où le calcul du spectre 2SD comprend :

le calcul d'un spectre d'incertitude totale par somme du spectre d'incertitude épistémique et du spectre d'incertitude aléatoire ;
le calcul d'un spectre d'écart standard (spectre SD) à partir d'une demi-puissance du spectre d'incertitude totale ; et
le calcul du spectre 2SD par multiplication par 2 du spectre SD.

**7.** Procédé selon la revendication 1, comprenant en outre
la conversion de la valeur de teneur en métabolites normalisée et de la valeur d'écart standard des métabolites normalisée sous forme de vecteur et la transmission du vecteur converti à une unité de sortie (150, 152, 154, 340).

**8.** Procédé selon la revendication 1 ou la revendication 7, comprenant en outre :

la formation d'un signal reconstruit par calcul d'une différence entre la valeur de teneur en métabolites normalisée et le spectre moyen prédictif ;
la formation d'un signal reconstruit par calcul d'une différence entre la valeur d'écart standard normalisée des métabolites et le spectre 2SD ; et
le contrôle de précision de la normalisation sur la base d'un résultat de comparaison des signaux reconstruits.

**9.** Procédé selon l'une des revendications 1 à 8, comprenant en outre la transmission du spectre moyen prédictif et le spectre 2SD à une unité de sortie (150, 152, 154, 340).

**10.** Appareil de quantification (300) pour la quantification de métabolites dans le corps, comprenant :

un ou plusieurs processeurs (21) ; et
une ou plusieurs mémoires (25) prévues pour stocker des instructions dont l'exécution par le ou les processeurs entraîne ledit ou lesdits processeurs à effectuer un calcul, ledit calcul effectué par ledit ou lesdits processeurs (21) comprenant :

a) un calcul de pré-traitement de données cryptées de spectroscopie par résonance magnétique (MRS) (S410-S470) afin d'obtenir un spectre d'entrée ;
b) un calcul de prévision (S520) d'informations d'incertitude aléatoire et d'un spectre d'échantillon à partir du spectre d'entrée pré-traité au moyen d'un réseau neuronal artificiel ;
c) un calcul (S580, S590) d'un spectre moyen prédictif du spectre d'échantillon prédit et d'un spectre d'écart à deux normes, 2SD, du spectre d'échantillon prédit sur la base du spectre d'échantillon prédit et des informations d'incertitude aléatoire prédites ;
d) un calcul d'exécution d'une correction de ligne de base sur le spectre moyen prédictif et le spectre 2SD ;
e) un calcul (S612) d'une valeur de teneur en métabolites par exécution (S640) d'une régression linéaire sur le spectre moyen prédictif corrigé ;
f) un calcul (S622) d'une valeur d'écart standard des métabolites par exécution (S640) d'une régression linéaire sur le spectre 2SD corrigé ; et
g) un calcul de normalisation (S614, S624) de la valeur de teneur en métabolites ainsi que de la valeur d'écart standard des métabolites ;
h) où le calcul de normalisation est basé sur (1) une teneur relative des métabolites par rapport à l'eau ou (2) une teneur relative des métabolites par rapport à une métabolite de référence ;
i) où la teneur relative des métabolites par rapport à l'eau est calculée sur la base de l'équation 1,

[Equation 1]

$$Conc_{meta} = 55 * \left(\frac{S_{meta}}{S_{water}}\right) * CorrTiss * \frac{Nspins_{water}}{Nspins_{meta}},$$

où $Conc_{meta}$ désigne une teneur d'une métabolite cible (mmol/L),

$S_{meta}$ désigne une zone de signal de la métabolite cible,
$S_{water}$ désigne une zone de signal de l'eau,
CorrTiss désigne un rapport de l'eau dans un tissu biologique dont un signal de spectroscopie par résonance magnétique (MRS) est obtenu,
$Nspins_{water}$ désigne le nombre de spins d'eau participant à la génération d'un signal de résonance, et
$Nspins_{meta}$ désigne le nombre de spins de la métabolite cible participant à la génération du signal de résonance.

**11.** Support d'enregistrement non transitoire lisible par ordinateur, pour le stockage d'un programme informatique pour l'exécution du procédé de quantification selon l'une des revendications 1 à 9.

**FIG. 1**

~ 100

WIRELESS COMMUNICATION UNIT — 110

- 111 — BROADCAST RECEIVING MODULE
- 112 — MOBILE COMMUNICATION MODULE
- 113 — WIRELESS INTERNET MODULE
- 114 — NEAR FIELD COMMUNICATION MODULE
- 115 — LOCATION INFORMATION MODULE

INPUT UNIT — 120

- 121 — CAMERA
- 122 — MICROPHONE
- 123 — USER INPUT UNIT

SENSING UNIT — 140

- 141 — PROXIMITY SENSOR
- 142 — ILLUMINATION SENSOR

CONTROL UNIT — 180

OUTPUT UNIT — 150

- 151 — DISPLAY UNIT
- 152 — SOUND OUTPUT UNIT
- 153 — HAPTIC MODULE
- 154 — OPTICAL OUTPUT UNIT

INTERFACE UNIT — 160

MEMORY — 170

POWER SUPPLY UNIT — 190

**FIG. 2**

20

27

21

25

COMMUNICATION UNIT ← → AI PROCESSOR ← → MEMORY

# FIG. 3A

300

## FIG. 3B

**FIG. 4**

FIG. 5

EP 4 248 846 B1

```
                              ┌─────────┐
                              │  START  │
                              └────┬────┘
                                   │              S510
                         ┌─────────▼─────────┐
                         /    INPUT DATA      /
                         └─────────┬─────────┘
                                   │              S520
                    ┌──────────────▼──────────────┐
                    │  PREDICT SAMPLE SPECTRUM AND │
                    │ ALEATORIC UNCERTAINTY        │
                    │ INFORMATION                  │
                    └──┬────────────────────────┬──┘
       S530            │                        │            S540
   ┌────────▼────────┐                    ┌─────▼─────────────────────────┐
   │ TEMPORARILY STORE│                   │ TEMPORARILY STORE             │
   │ SAMPLE SPECTRUM  │                   │ ALEATORIC UNCERTAINTY         │
   │                  │                   │ INFORMATION                   │
   └────────┬─────────┘                   └───────────────┬───────────────┘
            │                    S560                      │            S570
 ┌──────────▼──────┐  ┌──────────▼──────────────┐  ┌───────▼────────────────────┐
 │ CALCULATE AVERAGE│  │ CALCULATE VARIANCE      │  │ CALCULATE AVERAGE          │
 │          S550    │  │ (EPISTEMIC UNCERTAINTY  │  │ (ALEATORIC UNCERTAINTY     │
 │                  │  │  SPECTRUM)              │  │  SPECTRUM)                 │
 └──────────┬───────┘  └─────────────┬──────────┘  └────────────┬───────────────┘
            │      S580               │                          │          S590
 ┌──────────▼───────┐                 │           ┌──────────────▼──────────────┐
 │ CALCULATE        │                 └──────────►│ CALCULATE                   │
 │ PREDICTIVE MEAN  │                             │ TWO-STANDARD DEVIATION      │
 │ SPECTRUM         │                             │ SPECTRUM                    │
 └──────────┬───────┘                             └──────────────┬──────────────┘
            │                                                     │
            └─────────────────────────┬───────────────────────────┘
                                  ┌───▼───┐
                                  │  END  │
                                  └───────┘
```

FIG. 6

EP 4 248 846 B1

**FIG. 7**

**FIG. 8**

```
                         ┌──────────┐
                         │  START   │
                         └────┬─────┘
                              │
┌─────────────────────────────────────────────────────────────────┐      S8010
│   RECEIVE ENCRYPTED MRS DATA, PRE-PROCESS RECEIVED ENCRYPTED MRS  │
│     DATA, AND TRANSMIT PRE-PROCESSED DATA TO PREDICTION UNIT      │
└─────────────────────────────┬───────────────────────────────────┘
                              │
┌─────────────────────────────────────────────────────────────────┐      S8020
│  PREDICT SAMPLE SPECTRUM AND ALEATORIC UNCERTAINTY INFORMATION    │
│          OF MRS DATA BASED ON PRE-PROCESSED DATA                  │
└─────────────────────────────┬───────────────────────────────────┘
                              │
┌─────────────────────────────────────────────────────────────────┐      S8030
│  CALCULATE AVERAGE OF SAMPLE SPECTRA TO GENERATE PREDICTIVE MEAN  │
│                            SPECTRUM                               │
└─────────────────────────────┬───────────────────────────────────┘
                              │
┌─────────────────────────────────────────────────────────────────┐      S8040
│   CALCULATE EPISTEMIC UNCERTAINTY SPECTRUM OF SAMPLE SPECTRA      │
│            THROUGH VARIANCE OF SAMPLE SPECTRA                     │
└─────────────────────────────┬───────────────────────────────────┘
                              │
┌─────────────────────────────────────────────────────────────────┐      S8050
│    CALCULATE ALEATORIC UNCERTAINTY SPECTRUM OF SAMPLE SPECTRA     │
│       THROUGH AVERAGE OF ALEATORIC UNCERTAINTY INFORMATION        │
└─────────────────────────────┬───────────────────────────────────┘
                              │
┌─────────────────────────────────────────────────────────────────┐      S8060
│               CALCULATE TWO-STANDARD DEVIATION SPECTRUM           │
│  BASED ON EPISTEMIC UNCERTAINTY SPECTRUM AND ALEATORIC            │
│                   UNCERTAINTY SPECTRUM                            │
└─────────────────────────────┬───────────────────────────────────┘
                              │
                         ┌────┴─────┐
                         │   END    │
                         └──────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HYEONGHUN LEE AND HYEONJIN KI.** Bayesian deep learning-based 1H-MRS of the brain: Metabolite quantification with uncertainty estimation using Monte Carlo dropout. *PROCEEDINGS OF THE 2021 ISMRM & SMRT ANNUAL MEETING & EXHIBITION,* 30 April 2021, (2014